# EUROPEAN PATENT SPECIFICATION

(11) **EP 0 585 083 B1**
(45) Date of publication and mention of the grant of the patent: **13.12.1995**
(21) Application number: 93306628.4
(22) Date of filing: 20.08.1993
(51) Int. Cl.: C12N 15/54, C12N 9/10

(54) **Human glycosyltransferase gene**
Menschliches Glycosyltransferase Gen
Gène de glycosyltransferase humain

(30) Priority: 21.08.1992 JP 243984/92
(43) Date of publication of application: 02.03.1994
(73) Proprietor: TAKARA SHUZO CO. LTD., Fushimi-ku Kyoto 612 (JP)
(72) Inventor: Nishikawa, Atsushi, Toyonaka-shi, Osaka-fu (JP); Taniguchi, Naoyuki, Toyonaka-shi, Osaka-fu (JP)
(74) Representative: Wakerley, Helen Rachael

(56) References cited:
- WO-A-92/09694
- J. BIOCHEM. vol. 113, no. 6, June 1993, TOKYO pages 692 - 698 Y.IHARA ET AL. 'cDNA cloning, expression and chromosomal localisation of human GnT-III'
- J. BIOL. CHEM. vol. 267, no. 25, 5 September 1992, pages 18199 - 18204 NISHIKAWA A. ET AL. 'Purification, cDNA cloning and expression of GnT-III from rat kidney'
- CLIN. CHIM. ACTA vol. 185, no. 3, 15 December 1989, pages 325 - 332, ISHIBASHI, K ET AL. 'GnT-III in human serum and liver and hepatoma tissues...'
- BIOCHEM. BIOPHYS. RES. COMMUN. vol. 172, no. 3, 15. November 1990, pages 1260 - 1266, NAKAO, H ET AL. "Modulation of Gnt. III, IV, V activities and ..."

## Description

This invention relates to a human glycosyltransferase gene and a process for producing the transferase which is useful in the field of sugar engineering.

UDP-N-acetylglucosamine:β-D-mannoside β1-4N-acetylglucosaminyltransferase III (EC 2.4.1.144: hereinafter referred to simply as GnT-III), which is an enzyme transferring a GlcNAc residue in UDP-N-acetylglucosamine (UDP-GlcNAc) to a mannose (Man) residue forming a β1-4 bond in an asparagine binding type sugar chain, was reported for the first time by Narasimhan [see Journal of Biological Chemistry, 257, 10235-10242 (1982)]. The GlcNAc transferred by GnT-III, which is called a bisecting GlcNAc, has been found out in the sugar chains of various glycoproteins. It has been further reported by Narasimhan et al. [see J. Biol. Chem., 263, 1273-1282 (1988)] and Pascale et al. [see Carcinogenesis, 10, 961-964 (1989)] that the activity of GnT-III increases in rat liver accompanying its canceration.

In addition, Ishibashi et al. have reported an increase in the activity of GnT-III in the serum of a human patient with hepatic cancer [see Clinica Chimica Acta, 185, 325-332 (1989].

Regarding genes, furthermore, a gene coding for GnT-III originating in rat (rat GnT-III) has been isolated by one of the present inventors [see Japanese Patent Application No. 69345/1992].

As described above, GnT-III plays an important role in vivo and is a highly useful enzyme in the diagnosis of cancer because its activity increases accompanying canceration. However reports on human GnT-III have been limited to the determination of its activity and there has been no report of isolation of a human GnT-III gene so far.

The present invention aims at specifying a human GnT-III gene and providing a genetic engineering process for producing human GnT-III.

The present invention provides an isolated gene as claimed in claim 1, a gene hybridizable therewith, a vector containing either gene, a transformed cell containing the vector and a method of producing human GnT III using the transformed cell.

The present invention can be summarized as follows. The present invention relates to a human glycosyltransferase gene. The present invention also relates to a process for producing a human glycosyltransferase.

The present inventors have prepared a probe from a rat GnT-III gene, screened a human cDNA library for clones containing a gene coding for human GnT-III by using this probe, and succeeded in the isolation of the gene coding for human GnT-III and the expression of human GnT-III with the use of this gene, thus completing the present invention.

Now the present invention will be described in detail.

Examples of the probe which can be used in the detection of a gene coding for human GnT-III include a DNA fragment of approximately 1.4 kb which is obtained by cleaving a plasmid SV3 containing the rat GnT-III gene described in the Japanese Patent Application No. 69345/1992 with a restriction enzyme HindIII. The plasmid SV3 can be prepared from Escherichia coli XL1-Blue SV3 (FERM BP-4325) which has been deposited with Fermentation Research Institute, Agency of Industrial Science and Technology. By using this DNA fragment as a probe, for example, a commercially available human cDNA library can be screened for a gene coding for human GnT-III by plaque hybridization. As the result of the screening, two positive clones are obtained from 3 x 10⁶ plaques and respectively named H2 and H3. These clones are digested with EcoRI and then subcloned into, for example, the EcoRI site of Bluescript IISK⁺ (Stratagene). These subcloned plasmids are named respectively pBluescript II (H2) and pBluescript II (H3).

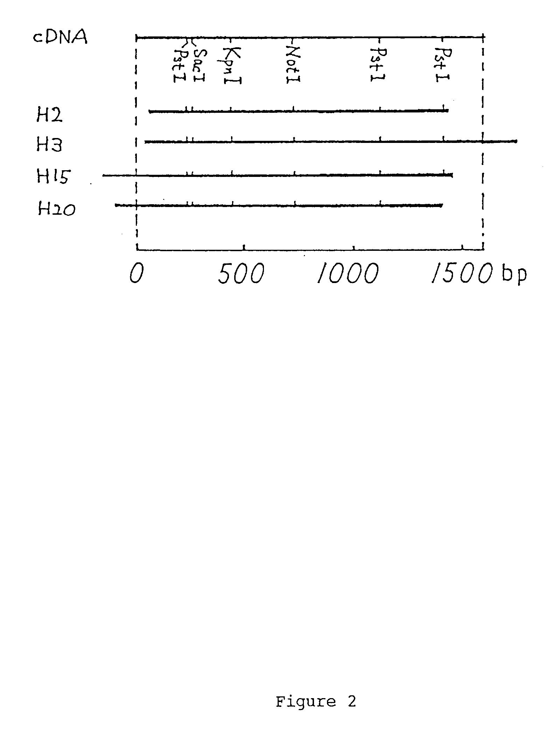

Figure 2 shows a relationship between H2 and H3 revealed by analyzing with restriction enzymes. Examination of the base sequences of these H2 and H3 reveals that no initiator codon ATG is involved therein. In order to obtain a full-length gene coding for human GnT-III, therefore, screening of the human cDNA library is effected again with the use of H2 and H3 as a probe. As the result, four positive clones are obtained from 7 x 10⁵ plaques. These clones are digested with EcoRI and then subcloned into, for example, the EcoRI site of Bluescript IISK⁺.

From among the subcloned DNAs, two DNAs containing an initiator codon are named respectively H15 and H20. The plasmids having H15 and H20 subcloned therein are named respectively pBluescript II (H15) and pBluescript II (H20). Figure 2 shows a relationship between H15 and H20 revealed by analyzing with restriction enzymes.

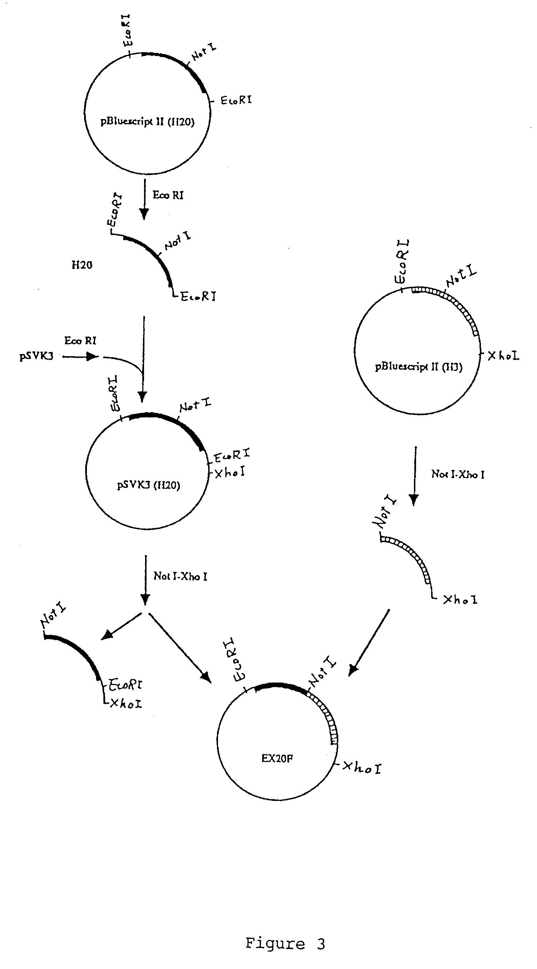

Next, an expression plasmid containing a full-length human GnT-III gene can be constructed by, for example, excising H20 from pBluescirpt II (H20) with EcoRI and integrating it into pSVK3 (Pharmacia) to prepare pSVK(H20). Further, a fragment excised from pBluescript II (H3) having H3 integrated thereinto with NotI and XhoI is integrated into the pSVK(H20) in such a manner as to replace the NotI-XhoI fragment of the pSVK(H20) therewith, thus constructing an expression plasmid EX20F (see Fig. 3).

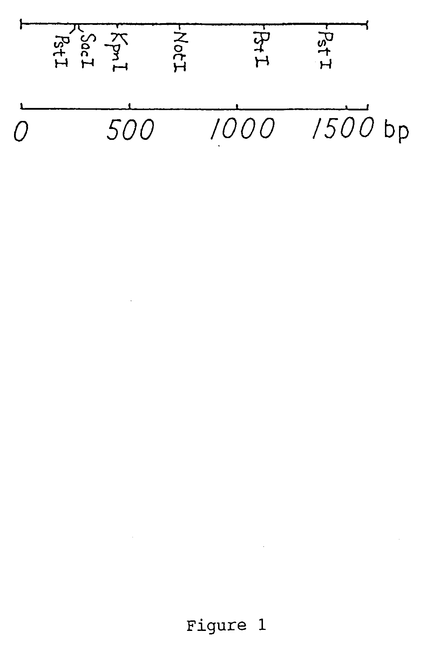

The base sequence of the DNA fragment integrated into EX20F can be determined by the dideoxy method. SEQ ID NO. 1 in the sequence listing shows a part of the base sequence thus determined. As the result of screening of an open reading frame (ORF), an ORF is found out in bases Nos. 169 to 1761. Figure 1 shows a restriction map of this ORF region. SEQ ID NO. 2 in the sequence listing shows the base sequence and the deduced amino acid sequence of the ORF region.

Human GnT-III producing cells can be obtained by transforming expression cells by an expression plasmid and examining the capability of the transformant to express human GnT-III. Examples of usable expression cells include COS-1 cells (ATCC CRL 1650). For example, the COS-1 cells can be transformed by the above expression plasmid EX20F. Then the transformant is incubated and the activity of GnT-III expressed in the transformant is determined to specify a gene coding for human GnT-III. This gene is integrated into EX20F and a part of its base sequence is located on a DNA fragment represented by SEQ ID NO. 1. Human GnT-III can be produced by genetic engineering technique by incubating the above transformant.

By effecting hybridization with the use of the gene thus obtained as a probe under strict conditions, it is anticipated that genes for enzymes analogous to that of the present invention, which are different therefrom in sequence but expected to have a similar activity, may be obtained. The term "under stringent conditions" as used herein means that the hybridization of a nylon membrane having DNAs immobilized thereon with the probe is conducted in a solution containing 6 x SSC (1 x SSC means a solution prepared by dissolving 8.76 g of sodium chloride and 4.41 g of sodium citrate in 1 liter of water), 1% of sodium lauryl sulfate, 100 »g/ml of salmon sperm DNA, and 5 x Denhardt's (containing bovine serum albumin, polyvinylpyrrolidone and Ficoll each at a concentration of 0.1%) at 65°C for 20 hours.

As described above in detail, the present invention enables a gene coding for human GnT-III to be isolated and provides a process for producing human GnT-III by using the gene. This gene and its decomposition products are usable in the determination of human GnT-III during the expression process in vivo and, therefore, useful in the genetic diagnosis of cancer, and so forth. In addition, various antibodies can be immunologically prepared by using polypeptides coded for by the gene of the present invention. These antibodies are also useful in the field of diagnosis and for the purification of human GnT-III.

### Brief Description of the Drawings

Figure 1 is a drawing showing a restriction map of a gene coding for human GnT-III. Figure 2 is a drawing showing relationships among four DNAs H2, H3, H15 and H20. Figure 3 is a drawing showing the construction of a plasmid EX20F.

### Example

To further illistrates the present invention in greater detail, and not by way of limitation, the following Examples will be given.

### Example 1

### (1) Screening of cDNA library

SV3 was prepared from Escherichia coli XL1-Blue SV3 (FERM BP-4325) transformed by a plasmid SV3 and the plasmid was digested with HindIII to give a DNA fragment of approximately 1.4 kb. This DNA fragment was radiolabeled with [α-³²P] dCTP (3000 Ci/mmol, Amersham) by using a Multiprime DNA Labeling System (Amersham) to thereby give a probe. By using the obtained probe, a human cDNA library [Human Fetal Liver 〈λgt10〉, Clonetech] was screened for the target clone by plaque hybridization. As a result, two positive clones were obtained from 3 x 10⁶ plaques. From these clones, DNAs were extracted and digested with EcoRI. The digestion products thus obtained were subcloned into Bluescript IISK⁺ and the DNAs thus subcloned (approximately 1.3 kb and approximately 1.5 kb) were respectively named H2 and H3, while the plasmids were respectively named pBluescript II (H2) and pBluescript II (H3). Figure 2 shows the restriction maps of these DNAs and a relationship between them.

### (2) Cloning of upstream region containing initiator codon

H2 and H3 were radiolabeled in the same manner as the one described in the above Example 1-(1) to thereby give probes. By using these probes, screening of a human cDNA library was carried out in the same manner as the one described in the above Example 1-(1) to obtain four positive clones from 7 x 10⁵ plaques. The EcoRI-digestion products thereof were subcloned into Bluescript IISK⁺. The base sequences of the DNAs thus subcloned were identified and two DNAs containing an initiator codon (approximately 1.6 kb and approximately 1.5 kb) were named respectively H15 and H20, while the plasmids corresponding thereto were named respectively pBluescript II (H15) and pBluescript II (H20). Figure 2 shows the restriction maps of these DNAs and a relationship between thereof with H2 and H3.

### Example 2

### (1) Construction of expression plasmid

The pBluescript II (H20) prepared in the above Example 1-(2) was digested with EcoRI to excise H20, which was integrated into the EcoRI site of an expression vector pSVK3 (Pharmacia) for eukaryotic cells to thereby give pSVK(H20). Separately, the pBluescript II (H3) prepared in the above Example 1-(1) was digested with NotI and XhoI to obtain an NotI-XhoI fragment (approximately 0.9 kb) containing the downstream region of the human GnT-III gene. Next, an expression plasmid EX20F for eukaryotic cells was constructed by digesting pSVK(H20) with NotI and XhoI, excising the NotI-XhoI fragment away, and integrating the NotI-XhoI fragment (approximately 0.9 kb) of pBLuescript II (H3) in its place (see Fig. 3).

The base sequence of the DNA fragment integrated into EX20F was determined by the dideoxy method. A part of this base sequence is represented by SEQ ID NO. 1 in the sequence listing. As the result of the screening for an ORF, an ORF was found in the bases Nos. 169 to 1761. Figure 1 shows a restriction map of this ORF region. SEQ ID NO. 2 in the sequence listing shows the base sequence and the deduced amino acid sequence of the ORF region.

### (2) Transformation and expression of human GnT-III gene

The COS-1 cells to be used for expressing human GnT-III were incubated in Dulbecco's modified Eagle medium containing 10% of FCS in the presence of 5% of CO₂ at 37°C under moist conditions.

Subsequently, the EX20F prepared in the above Example 2-(1) was introduced into the COS-1 cells by electropolation with the use of a Gene Pulser (Bio-Rad). More specifically, approximately 5 x 10⁶ cells and a recombinant plasmid or a control vector were suspended in 0.8 ml of 20 mM Hepes buffer (pH 7.05) containing 137 mM of NaCl, 5 mM of KCl, 0.7 mM of Na₂HPO₄ and 6 mM of dextrose, and treated at a voltage of 250 V/0.4 cm and at a capacitance of 960 »FD.

After the completion of the transformation, the incubation was carried out for 2 days and then the cells were harvested and sonicated in PBS to determine the GnT-III activity in the sonicated cell suspension. It was found that the cells which had not been treated with the plasmid and those which had been treated with the control plasmid pSVK3 had no GnT-III activity, whereas the cells transformed by the plasmid EX20F had a GnT-III activity, suggesting that the human GnT-III gene had been expressed therein.

Thus it has been proved that a gene coding for human GnT-III exists on the DNA fragment which have been integrated into EX20F and a part of the base sequence of which is represented by SEQ ID NO. 1 in the sequence listing.

### (3) Determination of GnT-III activity

The GnT-III activity was determined in accordance with the description given in Biochimica et Biophysica Acta, 1035, 313-318 (1990). More precisely, by using 80 »M of pyridylamino (PA) Gn, Gn-bi [GlcNAc β1-2Man α1-6 (GlcNAc β1-2Man α1-3)Man β1-4 GlcNAc β1-4 GlcNAc-PA] as a receptor, UDP-GlcNAc serving as a sugar donor was added in such a manner as to give a final concentration of 80 »M to a 125 mM solution of 2-(N-morpholino)ethanesulfonic acid buffer (MES buffer, pH 6.25) containing 10 mM of MnCl₂, 200 mM of GlcNAc and 0.5% (v/v) of Triton X-100. After reacting the mixture at 37°C for 1 hour, the GnT-III activity was determined by analyzing by HPLC.

### Effects of the Invention

According to the present invention, a gene for human GnT-III having an important role in vivo and an industrial process for producing this enzyme are provided. The gene and enzyme are useful in the fields of, for example, biochemistry and diagnosis.

Sequence Listing

SEQ ID NO:1

LENGTH:2247

TYPE:nucleic acid

STRANDEDNESS:double

TOPOLOGY:linear

MOLECULE TYPE:cDNA to mRNA

SEQUENCE DESCRIPTION:SEQ ID NO:1:

CCGGCTGCGA TGCCGGGCGC CCGCCGCAGC CGCTGCCGCC GGAGCCCGGG ATGGGGCGAG 60

AGGCTGCGGC GGACGCCAGC ATCTCCCCGC CGGGGACCCC GGGGGCCGCG GAGCCGCCGC 120

CGCCGCTGCT GCCGCCGTTG CTGAGACCCA GCGGGCGATG GGATGAAGAT GAGACGCTAC 180

AAGCTCTTTC TCATGTTCTG TATGGCCGGC CTGTGCCTCA TCTCCTTCCT GCACTTCTTC 240

AAGACCCTGT CCTATGTCAC CTTCCCCCGA GAACTGGCCT CCCTCAGCCC TAACCTGGTG 300

TCCAGCTTTT TCTGGAACAA TGCCCCGGTC ACGCCCCAGG CCAGCCCCGA GCCAGGAGGC 360

CCTGACCTGC TGCGTACCCC ACTCTACTCC CACTCGCCCC TGCTGCAGCC GCTGCCGCCC 420

AGCAAGGCGG CCGAGGAGCT CCACCGGGTG GACTTGGTGC TGCCCGAGGA CACCACCGAG 480

TATTTCGTGC GCACCAAGGC CGGCGGCGTC TGCTTCAAAC CCGGCACCAA GATGCTGGAG 540

AGGCCGCCCC CGGGACGGCC GGAGGAGAAG CCTGAGGGGG CCAACGGCTC CTCGGCCCGG 600

CGGCCACCCC GGTACCTCCT GAGCGCCCGG GAGCGCACGG GGGGCCGAGG CGCCCGGCGC 660

AAGTGGGTGG AGTGCGTGTG CCTGCCCGGC TGGCACGGAC CCAGCTGCGG CGTGCCCACT 720

GTGGTGCAGT ACTCCAACCT GCCCACCAAG GAGCGGCTGG TGCCCAGGGA GGTGCCGCGC 780

CGCGTCATCA ACGCCATCAA CGTCAACCAC GAGTTCGACC TGCTGGACGT GCGCTTCCAC 840

GAGCTGGGCG ACGTGGTGGA CGCCTTTGTG GTGTGCGAGT CCAACTTCAC GGCTTATGGG 900

GAGCCGCGGC CGCTCAAGTT CCGGGAGATG CTGACCAATG GCACCTTCGA GTACATCCGC 960

CACAAGGTGC TCTATGTCTT CCTGGACCAC TTCCCGCCCG GCGGCCGGCA GGACGGCTGG 1020

ATCGCCGACG ACTACCTGCG CACCTTCCTC ACCCAGGACG GCGTCTCGCG GCTGCGCAAC 1080

CTGCGGCCCG ACGACGTCTT CATCATTGAC GATGCGGACG AGATCCCGGC CCGTGACGGC 1140

GTCCTTTTCC TCAAGCTCTA CGATGGCTGG ACCGAGCCCT TCGCCTTCCA CATGCGCACG 1200

TCGCTCTACG GCTTCTTCTG GAAGCAGCCG GGCACCCTGG AGGTGGTGTC AGGCTGCACG 1260

GTGGACATGC TGCAGGCAGT GTATGGGCTG GACGGCATCC GCCTGCGCCG CCGCCAGTAC 1320

TACACCATGC CCAACTTCAG ACAGTATGAG AACCGCACCG GCCACATCCT GGTGCAGTGG 1380

TCGCTGGGCA GCCCCCTGCA CTTCGCCGGC TGGCACTGCT CCTGGTGCTT CACGCCCGAG 1440

GGCATCTACT TCAAGCTCGT GTCCGCCCAG AATGGCGACT TCCCACGCTG GGGTGACTAC 1500

GAGGACAAGC GGGACCTGAA CTACATCCGC GGCCTGATCC GCACCGGGGG CTGGTTCGAC 1560

GGCACGCAGC AGGAGTACCC GCCTGCAGAC CCCAGCGAGC ACATGTATGC GCCCAAGTAC 1620

CTGCTGAAGA ACTACGACCG GTTCCACTAC CTGCTGGACA ACCCCTACCA GGAGCCCAGG 1680

AGCACGGCGG CGGGCGGGTG GCGCCACAGG GGTCCCGAGG GAAGGCCGCC CGCCCGGGGC 1740

AAACTGGACG AGGCGGAAGT CTAGAGCTGC ATGATCTGAT AGGGTTTGTG ACAGGGCGGG 1800

GGTGGCGGCG GCCCCTAGCG CTATCTCCCT GCCTCCTGCC GGCTCCTTGG TTCTTGAGGG 1860

GACCAGGAGT GGGTGGGGAG TGGGGGTGGG GCTAGGGTTT CCCTACTGAA GCCCTTGTGA 1920

TCAAGGGTCA GGCCTTTGAG CTCAGAAAAT ATCCCTCCTG TTGGGAGAGG GCGCAGGCCG 1980

TGACGTCTGG GTGGCCCTTA TGACTGCCAA GACTGCTGTG GCCAGGAGGT GCCACTGGAG 2040

TGTGCGTGGT GGTCCCTGGG TAGCGGGGGA GGGTAGGCAG GATTGGGGAA GAGAGCCTGC 2100

AGGATCTCAC CAGGCAGCCT CTGGGGGGTG GCCAGGCCGG AAAAAGCCCA CCATTTGGCA 2160

TCCCTGGGCC TTGGGCTCCG TGTGGGAGAC CGGCCTGCCA GGAGGACCCA GGGCTCTGTA 2220

AGTAGATGCA TTTGGGTCCA GGAGGAA 2247

SEQ ID NO:2

LENGTH:1593

TYPE:nucleic acid

STRANDEDNESS:double

TOPOLOGY:linear

MOLECULE TYPE:cDNA to mRNA

SEQUENCE DESCRIPTION:SEQ ID NO:2:

ATG AGA CGC TAC AAG CTC TTT CTC ATG TTC TGT ATG GCC GGC CTG 45



1               5                   10                  15

TGC CTC ATC TCC TTC CTG CAC TTC TTC AAG ACC CTG TCC TAT GTC 90

                20                  25                  30
ACC TTC CCC CGA GAA CTG GCC TCC CTC AGC CCT AAC CTG GTG TCC 135

                35                  40                  45
AGC TTT TTC TGG AAC AAT GCC CCG GTC ACG CCC CAG GCC AGC CCC 180

                50                  55                  60
GAG CCA GGA GGC CCT GAC CTG CTG CGT ACC CCA CTC TAC TCC CAC 225

                65                  70                  75
TCG CCC CTG CTG CAG CCG CTG CCG CCC AGC AAG GCG GCC GAG GAG 270

                80                  85                  90
CTC CAC CGG GTG GAC TTG GTG CTG CCC GAG GAC ACC ACC GAG TAT 315

                95                  100                 105
TTC GTG CGC ACC AAG GCC GGC GGC GTC TGC TTC AAA CCC GGC ACC 360

                110                 115                 120
AAG ATG CTG GAG AGG CCG CCC CCG GGA CGG CCG GAG GAG AAG CCT 405

                125                 130                 135
GAG GGG GCC AAC GGC TCC TCG GCC CGG CGG CCA CCC CGG TAC CTC 450

                140                 145                 150
CTG AGC GCC CGG GAG CGC ACG GGG GGC CGA GGC GCC CGG CGC AAG 495


                155                 160                 165
TGG GTG GAG TGC GTG TGC CTG CCC GGC TGG CAC GGA CCC AGC TGC 540

                170                 175                 180
GGC GTG CCC ACT GTG GTG CAG TAC TCC AAC CTG CCC ACC AAG GAG 585

                185                 190                 195
CGG CTG GTG CCC AGG GAG GTG CCG CGC CGC GTC ATC AAC GCC ATC 630

                200                 205                 210
AAC GTC AAC CAC GAG TTC GAC CTG CTG GAC GTG CGC TTC CAC GAG 675

                215                 220                 225
CTG GGC GAC GTG GTG GAC GCC TTT GTG GTG TGC GAG TCC AAC TTC 720

                230                 235                 240
ACG GCT TAT GGG GAG CCG CGG CCG CTC AAG TTC CGG GAG ATG CTG 765

                245                 250                 255
ACC AAT GGC ACC TTC GAG TAC ATC CGC CAC AAG GTG CTC TAT GTC 810

                260                 265                 270
TTC CTG GAC CAC TTC CCG CCC GGC GGC CGG CAG GAC GGC TGG ATC 855

                275                 280                 285
GCC GAC GAC TAC CTG CGC ACC TTC CTC ACC CAG GAC GGC GTC TCG 900

                290                 295                 300
CGG CTG CGC AAC CTG CGG CCC GAC GAC GTC TTC ATC ATT GAC GAT 945


                305                 310                 315
GCG GAC GAG ATC CCG GCC CGT GAC GGC GTC CTT TTC CTC AAG CTC 990

                320                 325                 330
TAC GAT GGC TGG ACC GAG CCC TTC GCC TTC CAC ATG CGC ACG TCG 1035

                335                 340                 345
CTC TAC GGC TTC TTC TGG AAG CAG CCG GGC ACC CTG GAG GTG GTG 1080

                350                 355                 360
TCA GGC TGC ACG GTG GAC ATG CTG CAG GCA GTG TAT GGG CTG GAC 1125

                365                 370                 375
GGC ATC CGC CTG CGC CGC CGC CAG TAC TAC ACC ATG CCC AAC TTC 1170

                380                 385                 390
AGA CAG TAT GAG AAC CGC ACC GGC CAC ATC CTG GTG CAG TGG TCG 1215

                395                 400                 405
CTG GGC AGC CCC CTG CAC TTC GCC GGC TGG CAC TGC TCC TGG TGC 1260

                410                 415                 420
TTC ACG CCC GAG GGC ATC TAC TTC AAG CTC GTG TCC GCC CAG AAT 1305

                425                 430                 435
GGC GAC TTC CCA CGC TGG GGT GAC TAC GAG GAC AAG CGG GAC CTG 1350

                440                 445                 450

AAC TAC ATC CGC GGC CTG ATC CGC ACC GGG GGC TGG TTC GAC GGC 1395

                455                 460                 465
ACG CAG CAG GAG TAC CCG CCT GCA GAC CCC AGC GAG CAC ATG TAT 1440

                470                 475                 480
GCG CCC AAG TAC CTG CTG AAG AAC TAC GAC CGG TTC CAC TAC CTG 1485

                485                 490                 495
CTG GAC AAC CCC TAC CAG GAG CCC AGG AGC ACG GCG GCG GGC GGG 1530

                500                 505                 510
TGG CGC CAC AGG GGT CCC GAG GGA AGG CCG CCC GCC CGG GGC AAA 1575
 525
                515                 520                 525
CTG GAC GAG GCG GAA GTC 1593

                530

## Claims

1. Isolated gene encoding human glycosyltransferase III (GnT-III), wherein the nucleotide sequence is as shown by SEQ ID NO. 2 in the sequence listing, or a derivative thereof by way of nucleic acid deletion, substitution, insertion, inversion, addition or replacement.

2. Isolated gene encoding human GnT-III which is hybridizable with the gene of claim 1 under stringent conditions.

3. A vector which contains the isolated gene of claim 1 or 2.

4. A transformed cell which contains the vector of claim 3.

5. A method of producing human GnT-III which comprises cultivating transformed cells of claim 4 and isolating human GnT-III from the culture medium in which the transformed cells have been cultivated.

## Patentansprüche

1. Isoliertes Gen, welches die menschliche Glycosyltransferase III (GnT-III) kodiert, wobei die Nukleotidsequenz so ist, wie sie von SEQ ID Nr. 2 im Sequenzprotokoll gezeigt ist, oder ein Derivat davon, und zwar mittels Nukleinsäuredeletion, -Substitution, -Insertion, -Inversion, -Addition oder -Ersatz.

2. Isoliertes Gen, welches die menschliche GnT-III kodiert, welches unter strikten Bedingungen mit dem Gen von Anspruch 1 hybridisierbar ist.

3. Vektor, welcher das isolierte Gen von Anspruch 1 oder Anspruch 2 enthält.

4. Transformierte Zelle, welche den Vektor von Anspruch 3 enthält.

5. Verfahren zur Herstellung von menschlicher GnT-III, bei welchem Verfahren transformierte Zellen von Anspruch 4 kultiviert werden und menschliche GnT-III aus dem Kulturmedium, in welchem die transformierten Zellen kultiviert wurden, isoliert wird.

## Revendications

1. Gène isolé codant pour la glycosyltransférase III (GnT-III) humaine, dans lequel la séquence de nucléotides est telle que représentée par la SEQ ID N° 2 dans la liste des séquences, ou un de ses dérivés engendrés par délétion, substitution, insertion, inversion, addition ou remplacement d'acide nucléique.

2. Gène isolé codant pour la GnT-III humaine, qui est hybridable avec le gène suivant la revendication 1 dans des conditions drastiques.

3. Vecteur qui contient le gène isolé suivant la revendication 1 ou la revendication 2.

4. Cellule transformée qui contient le vecteur suivant la revendication 3.

5. Procédé de production de GnT-III humaine, qui comprend la culture de cellules transformées suivant la revendication 4 et l'isolement de GnT-III humaine du milieu de culture dans lequel les cellules transformées ont été cultivées.
